# EUROPÄISCHE PATENTANMELDUNG

(11) **EP 3 485 898 A1**
(43) Veröffentlichungstag der Anmeldung: **22.05.2019**
(21) Anmeldenummer: 17020539.7
(22) Anmeldetag: 20.11.2017
(51) Int. Cl.: A61K 38/18, A61K 39/00, A61K 35/15, C12N 5/0784

(54) **VERFAHREN ZUM SCHONENDEN ZELLAUFSCHLUSS VON TUMORZELLEN DURCH ULTRAKURZPULS-LASERSTRAHLUNG UND EINER DARAUF BASIERENDEN IMMUNAKTIVIERUNG**

(71) Anmelder: mfd Diagnostics GmbH, 55234 Wendelsheim (DE)
(72) Erfinder: Lecher, Bernd, 55128 Mainz (DE); Maurer-Vogt, Simone, 68 723 Schwetzingen (DE); Kempe, Christian, 14469 Potsdam (DE)
(74) Vertreter: Kayser, Christoph

(57) **Zusammenfassung**

Ein Verfahren zur Aktivierung von aus Patientenblut gewonnenen Immunzellen zur Stimulation einer antitumoralen körpereigenen Immunantwort, ist gekennzeichnet durch die Schritte:
- Perforieren der Zellmembran von Tumorzellen mittels eines Ultrakurzpuls-Lasers zur Gewinnung von nativen Tumorantigenen und Teilen der perforierten Zellmembran;
- Kokultivieren von aus Patientenblut gewonnen Immunzellen mit den nativen Tumorantigenen und Teilen der perforierten Zellmembran.

## Beschreibung

Die vorliegende Erfindung betrifft ein Verfahren zur Aktivierung von aus Patientenblut gewonnenen Immunzellen zur Stimulation einer antitumoralen körpereigenen Immunantwort.

Konventionelle Strategien zur Gewinnung von Zellbestandteilen aus lebenden Zellen eines Zellverbundes nutzen vorwiegend mechanische und chemischbiochemische, aber auch thermische und elektrostatische Aufschlussmechanismen. Die wichtigsten Methoden werden im Folgenden kurz beschrieben:

### Aufschluss durch Scherkräfte

### a. Potter-Elvehjem Verfahren

| | |
|---|---|
| Prinzip: | Ein Kolben bewegt sich in einem stationären Gefäß das eng ummantelt ist. Die Zerstörung der Zellen erfolgt durch Scherkräfte. |
| Anwendung: | wenn die Zellorganellen erhalten bleiben sollen |
| Nachteile: | physikalischer Energieeintrag durch Wärmeentwicklung, mechanische Zerstörung der Zellmembran und der Strukturproteine, Zerstörung größerer Zellorganelle wie Mitochondrien |

### b. Zerstörung mittels Mörser oder Kugelmühle

| | |
|---|---|
| Prinzip: | mechanische Zerstörung der Zellen durch Druck, Reibung und Scherkräfte |
| Anwendung: | bei allen Anwendungen, bei denen die Integrität der Zellbestandteile (Zellorganellen, Zellmembran, Strukturproteine, langkettige Polymere und große Funktionsproteine) nicht erhalten bleiben muss |
| Nachteile: | großer mechanischer und thermischer Energieeintrag, der zur Zerstörung von Zellorganellen, Zellmembran, Strukturproteinen, langkettigen Polymere und großen Funktionsproteine. |

### Aufschluss durch Kavitationskräfte

### a. Ultraschallverfahren:

| | |
|---|---|
| Prinzip: | Der Zellaufschluss wird in dem Verfahren durch Kavitationskräfte bewerkstelligt. |
| Anwendung: | Das Verfahren wird häufig für sehr kleine Probenmengen eingesetzt. |
| Nachteile: | Lokal treten sehr hohe Temperaturen auf, die einerseits zu Strukturveränderungen der Zellorganellen führen und andererseits oft Einbußen in der Ausbeute verursachen. Außerdem wird die Integrität der Zell-Zell-Kontakte |
| | zerstört. Weitere Nachteile sind die Unmöglichkeit einer Skalierbarkeit und Lärmentwicklung. |

### b. PET-Verfahren (Pulsed Electric Fields)

| | |
|---|---|
| Prinzip: | Die Wirkung des Verfahrens beruht auf einer Elektroporation der Zellen. Die Zellen werden zwischen zwei Elektroden platziert und mit elektrischen Pulsen behandelt, die durch hohe Feldstärken (10 - 80 kV/cm) und eine kurze Dauer (500 ns bis 4 µs) charakterisiert sind. Dabei findet eine reversible oder irreversible Porenöffnung in den Zellmembranen statt, wodurch intrazelluläre Bestandteile freigesetzt werden können. |

### Aufschluss durch Druck

### a. French Press Verfahren

| | |
|---|---|
| Prinzip: | Es wird Hochdruck in einer Druckkammer erzeugt. Mittels eines manuellen Ventils wird die Druckkammer geöffnet. Der Druckabfall bewirkt wird den Zellaufschluss. |
| Nachteile: | Scale-up und reproduzierbare Ergebnisse sind nicht möglich. French Pressen müssen aufwändig nach jeder Probe gereinigt werden, das manuelle Betätigen der |
| | Ventile ist kraftaufwendig und die Handhabung nicht ungefährlich. |

### b. Microfluidizer

| | |
|---|---|
| Prinzip: | Im Microfluidizer wird die Zellsuspension unter hohem Druck durch einen Mikrokanal mit fester interner Geometrie geführt, in dem aufgrund von Scherkräften und Pralleffekten die Zellwände aufgebrochen werden. |
| Anwendung: | Der Microfluidizer wird bei Probenmengen von 1 ml bis zu mehreren 100 1/h eingesetzt. |
| Nachteile: | Der Einsatz von hohen Prozessdrücken zerstört die Struktur wichtiger Zellorganelle und damit auch ihre funktionellen Komponenten. |

### c. Hochdruckhomogenisator (HPH)

| | |
|---|---|
| Prinzip: | Hochdruckhomogenisatoren haben ein ähnliches Verfahrensprinzip wie die Microfluidizer-Technologie, mit einigen entscheidenden Unterschieden: die eingesetzten Pumpen unterscheiden sich von denen im Microfluidizer und statt einer Interaktionskammer mit fester optimierter Geometrie kommen Homogenisierventile zum Einsatz. |
| Nachteile: | Der Einsatz von hohen Prozessdrücken zerstört die Struktur wichtiger Zellorganelle und damit auch ihre funktionellen Komponenten. Weitere häufige Probleme treten bei der Kühlung, Reinigung und Verschleiß der Ventile sowie bei der Skalierbarkeit auf. Des Weiteren ist eine in der Regel eine enzymatische (Vor-) Behandlung notwendig. Die Zellsuspension wird mit Enzymen auf den nachfolgenden Aufschluss vorbereitet. |

### d. Stickstoff-Dekompressionsmethode

| | |
|---|---|
| Prinzip: | In diesem Verfahren wird in den Zellen entsprechend dem Henry'schen Gesetz Stickstoff bei höheren GasDrücken angereichert. Das Henry'schen Gesetz beschreibt das Löslichkeitsverhalten von flüchtigen Substanzen in einer Flüssigkeit und besagt, dass die Konzentration der flüchtigen Substanz in der Flüssigphase direkt proportional zum Partialdruck über der Lösung ist, d.h. dass unter erhöhtem Gas-Druck das Gas in die Zellen diffundiert und sich dort anreichert. Kommt es dann zu einer schlagartigen Druckentlastung kann das innerhalb der Zellen gelöste Gas nicht schnell genug entweichen es entstehen innerhalb der Zellen Gasbläschen. Damit entsteht ein hoherDruck auf die |
| | Zellmembran, der schließlich Platzen der Zelle führt, wodurch der Zellinhalt freigesetzt wird. |
| Anwendung: | Aufschluss von Tier- und Pflanzenzellen als auch von empfindlichen Bakterien. Darüber hinaus wird die Methode auch erfolgreich für die Ausschleusung von Viren aus befruchteten Eiern verwendet. Mit verschiedenen Methoden können die die Zellwände geschwächt werden (z.B.Verwendung von Muramidase). |
| Nachteile: | Da die Methode vor allem dem Zweck dient, Zellhomogenisate herzustellen, wird in Kauf genommen, dass die Zellintegrität mechanisch zerstört wird. Dasselbe gilt sowohl für die Zellorganelle, als auch für die Zellmembranstruktur und die Zell-Zellkontaktstellen. Die Zellen werden komplett zerstört und sind für Mikromanipulationen sowie für spektral-analytsche Verlaufsuntersuchungen von beispielweise biochemischen Vorgängen (Kinetiken) nicht mehr zugänglich. Komplexe zellphysiologische Vorgänge können nicht mehr lokal und zeitlich aufgelöst analysiert und gemessen werden. |

### Aufschluss durch Kristallisation (Freeze-Thaw)

| | |
|---|---|
| Prinzip: | Durch den zyklischen Wechsel von variierenden Temperaturen erfolgt ein wiederholtes Einfrieren und Auftauen (Freeze-Thaw). Dadurch werden die Zellwände bzw. -membranen der Zellen durch Perforation aufgrund von Scherkräften der entstehenden Eiskristalle aufgebrochen und zerstört. |
| Anwendung: | Das Verfahren wird häufig für sehr kleine Probenmengen eingesetzt. |
| Nachteile: | Ein wesentlicher Nachteil des Verfahrens ist die schlechte Reproduzierbarkeit. Außerdem kommt es durch die Behandlung mit hypotonischen Pufferlösungen zu einer hypotonen Lyse der Zellen. |

### Chemische Lyse

| | |
|---|---|
| Prinzip: | Zugesetzte Chemikalien (z.B. eine Mischung aus pH-Puffer, Protease-Inhibitoren und einer Detergenz zur Auflösung der Zellmembran) erweichen die Zellwände und brechen sie auf. |
| Anwendung: | Die chemische Lyse wird in erster Linie bei Zellen angewandt, die sich mechanisch nicht einfach |
| | aufbrechen lassen (z.B. Hefen). Bei nicht-pflanzlichen Zellen erfolgt die Zerstörung der Zellmembran mittels Phenol-Chloroform-Extraktion z.B. mit Trizol durchgeführt. Dabei wird durch Herauslösen der Membranlipide aus der Zellmembran ein Aufschluss durchgeführt. Diese ist aber in Anwesenheit von Guanidinium-Thiocyanat denaturierend. Die Autolyse beispielsweise bei Hefezellen wird mit Toluol durchgeführt, welches Löcher in der Zellmembran erzeugt. Durch Verwendung des Enzyms Zymolyase wird die Glucan-Zellwand zerstört, ebenso mittels Triton X-100. |
| Nachteile: | Die Methode zerstört die Zellmembran und damit die strukturelle Integrität der Zelle. Des Weiteren werden alle Moleküle, die einen Lipidanteil besitzen ebenfalls zerstört; damit wird ihre Funktionalität ausgeschaltet. Das betrifft nicht nur einzelne Moleküle sondern auch Zellorganellen wie z.B. Mitochondrien, die Kernmembranen sowie alle Speichervakuolen. Die strukturabhängige Funktionalität der Zelle als Ganzes, aber auch der einzelnen Bestandteile wird vollständig aufgehoben. Ein weiter Nachteil besteht darin, dass die Chemikalien die Skalierbarkeit der Methode stark |
| | einschränken. Die Kontamination mit Chemikalien ist oft unerwünscht. |

Der Erfindung liegt daher die Aufgabe zugrunde, ein Verfahren zur Tumorbekämpfung mittels einer effizienten antitumoralen Immunantwort zur Verfügung zu stellen.

Die Aufgabe wird erfindungsgemäß gelöst durch die Schritte:
- Perforieren wenigstens einer Zellmembran wenigstens einer Tumorzelle mittels eines Ultrakurzpuls-Lasers zur Gewinnung von nativen Tumorantigenen und Teilen der wenigstens einen perforierten Zellmembran;
- Kokultivieren wenigstens einer von aus Patientenblut gewonnen Immunzellen mit den nativen Tumorantigenen und Teilen der wenigstens einen perforierten Zellmembran.

Dadurch, dass durch das erfindungsgemäße Verfahren lediglich die Zellmembran der Tumorzellen perforiert wird, d.h. nur die Integrität der Membran zerstört wird, stirbt die Zelle in Form einer Nekrose ab und kann nicht mehr den programmierten Zelltod (Apoptose) einleiten. Deshalb können diese Bestandteile als stimulierende Kofaktoren wirken und eine Immunreaktion ähnlich eines lokalen Entzündungsprozesses auslösen. Dadurch wird das Entstehen einer Toleranzreaktion verhindert.

Bei dem erfindungsgemäßen Verfahren treten keine Risiken auf, die bei Modifikationen der Tumorzellen durch Viren zu erwarten sind. Zu diesen Risiken gehören u.a.:
- ineffiziente Infektion der Zellen
- ineffiziente Infektionsdosis
- unerwünscht freigesetzten Viren
- Nichtkalkulierbarkeit von Nebenwirkungen mit Recall-Antigenen (z.B. allergische bzw. hyperergische Reaktion mit dem Risiko eines S chock/Kreislaufversagen)

Grundlage des vorgestellten Verfahrens ist die Gewinnung von nativen Tumorantigenen und kostimulierenden Faktoren mittels eines Ultrakurzpuls-Lasers durch die Perforation der Zellmembran von aus Patientenmaterial gewonnenen Tumorzellen. Mit diesem Material soll durch Kokultivierung mit Immunzellen desselben Patienten eine effektive Immunantwort stimuliert werden, die speziell gegen diesen Tumor gerichtet ist. Die Immunzellen werden im Anschluss in die Blutbahn dieses Patienten infundiert.

Die Erfindung schlägt ein Verfahren vor, um folgende immuntherapeutische Strategie zur Aktivierung des Immunsystems und zur Bekämpfung eines Patienten spezifischen Tumors zu realisieren:
- Tumorzellen aus Patientenmaterial (Biopsiematerial, Blut oder Lymphknoten) werden gegebenenfalls mittels eines tumorspezifischen fluoreszierenden Markers markiert und in ein optisches Device verbracht.
- Die Tumorzellen werden mittels eines Ultrakurzpuls-Lasers derart bestrahlt, dass die Zellmembran perforiert wird. Dabei bleibt die chemische Struktur der Zellbestandteile größtenteils unverändert, so dass nach dem Vorgang die Zellbestandteile in nativer Form frei vorliegen. Dies stellt gegenüber herkömmlichen Zelllyseverfahren, einschließlich der Bestrahlung, eine essentielle erfinderische Neuerung dar, die insbesondere durch folgende Punkte gekennzeichnet ist:
   ∘ Im Gegensatz zu anderen Verfahren bleibt das Zytoplasma in allen seinen molekularen Bestandteilen, in seiner chemischen Struktur und seinen physiko-chemischen Eigenschaften unverändert erhalten.
   ∘ Substrate und Metaboliten des Zytoplasmas bleiben erhalten.
   ∘ Es wird lediglich die Grenze des zellulären Systems eröffnet und damit die vitale Integrität der Tumorzelle zerstört.
   ∘ Es kommt nicht, wie bei der Bestrahlung der Tumorzellen zu chromosomalen Aberrationen und anderen DNA- bzw. RNA-Schäden (Strangbrüche, Punktmutationen etc.).
   ∘ Es entstehen keine DNA Fragmente, wie sie für Apoptosen typisch sind, so dass das Immunsystem keine Toleranz entwickeln kann.
- Im Anschluss wird das Zellmaterial in eine sterile, abgeschlossene Reaktionskammer überführt, in der die Kokultivierung mit Immunzellen, das aus dem Patientenblut gewonnen wurde durchgeführt werden kann.
- Im nächsten Schritt wird ein Teil des Zellüberstandes entnommen und ein Zytokinpanel gemessen. Dies gibt einerseits Auskunft über die Effizienz der antitumoralen Immunstimulanz und andererseits werden Anzeichen einer möglichen fehlgeleiteten Immunreaktion detektiert, um das Risiko einer möglichen Autoimmunreaktion ausschließen zu können. Alternativ dazu oder als ergänzende Methode kann die Aktivierung der Immunzellen durch Fluoreszenzmarkierung der entsprechenden Oberflächenproteine und nachfolgende Fluoreszenzanalyse mittels Durchflusszytometrie ermittelt werden.
- Nach Abschluss dieser Maßnahme werden dem Patienten das Zellmaterial und die kokultivierten Immunzellen reinfundiert. Dies führt zu einer Immunreaktion, vergleichbar mit einer Entzündungsreaktion, die zur Zerstörung des Primärtumors und eventuellen Metastasen führt.
- Diese Maßnahmen können bei nicht ausreichenden bzw. zufriedenstellenden Ergebnissen wiederholt werden.

Im Vergleich zu gentechnischen Transfer-Verfahren, bei denen auch stark antigene Substanzen, wie Recall-Antigene oder Teile derselben, gentechnisch in die Tumorzellen eingebracht werden, so dass diese dann entsprechende Neo-Antigene exprimieren, ist das Verfahren relativ einfach, jederzeit und schnell durchführbar, preisgünstig, deutlich weniger riskant und mit wenig Aufwand auch in externen Dienstleistungslabors durchführbar und jederzeit wiederholbar.

### Tumorstrategien

Tumore entwickeln sich aus einzelnen entarteten Zellen, die jedoch in sich heterogen sind. Sie unterliegen ständig einem Selektionsdruck, der in verschiedener Form auf Tumore einwirkt (z. B. durch die Immunüberwachung, das Nährstoffangebot, die Sauerstoffversorgung, die durchgeführte Therapie: Chemotherapie/Bestrahlung, etc.) und der zur klonalen Selektion solcher Zellen führt, die einen Wachstums oder Überlebensvorteil gegenüber dem ursprünglichen Klon aufweisen.

Dabei entwickeln Tumore Strategien, die einen effektiven Zugang von Therapeutika einschließlich Abwehrzellen erschweren bzw. unmöglich machen:
1. Solide Tumore entwickeln ab einer bestimmten Größe eine eigene Gefäßversorgung, die zwar von normalen Gefäßen ihren Ausgang nehmen, sich aber von diesen Gefäßen unterscheiden. Sie befinden sich in der Regel in der Randzone der Tumoren und werden zum Zentrum hin immer stärker desorganisiert. D.h. die rheologischen Eigenschaften, ebenso wie die Blutdruck- und Strömungsverhältnisse unterscheiden sich wesentlich vom normalen Kapillarnetz. Daraus resultiert die deutlich schlechtere Sauerstoff- und Nährstoffversorgung des Tumors. Das bedeutet aber auch, dass Tumorzellen, die schlechter versorgt sind, auch einen in der Regel anaeroben Stoffwechsel zur Energieversorgung nutzen. Andererseits wird es durch die schlechten Strömungseigenschaften therapeutisch wirksamen Substanzen erschwert, in tiefere Schichten des Tumors zu gelangen. Die extrazelluläre Matrix im Zentrum des Tumors hat eine deutlich höhere Festigkeit, als das umgebende Gewebe. Deshalb kann sich die Gefäßentwicklung nicht wie die eines normalen Kapillarnetzes nach optimalen Strömungs- und damit Versorgungskriterien entwickeln.
2. Die teilweise nicht durchgängigen Gefäßwände von Tumorgefäßen stellen eine weitere Barriere dar. Da die normale Gefäßauskleidung mit funktionellen Endothelzellen nur lückenhaft vorhanden ist, sind die tumoreigenen Gefäßwände unterschiedlich und teilweise nicht permeabel. Tumore können Zellen des Immunsystems dazu anregen, Angiogenesefaktoren zu bilden, um somit Endothelzellen dazu anzuregen, neue Gefäße zu bilden.
3. Das Interstitium eines Tumors behindert die Diffusion von Molekülen und Zellen im Tumor wesentlich. Verantwortlich dafür ist die Härte der Textur des Interstitiums, die die Viskosität wesentlich verschlechtert, d.h. das Strömungsverhalten von Flüssigkeiten wird massiv verschlechtert. Es kommt zu einer Stase, wodurch die Diffusion ebenfalls eingeschränkt wird, da der Druckgradient entgegen der Diffusion verläuft.
4. Ein weiterer Umstand, der zur Behinderung von Zelleinwanderung und zu chemischen Veränderungen bei Molekülen führt, die zur Tumorbekämpfung eingesetzt werden, ist auf den relativ niedrige pH Wert im Tumorgewebe zurückzuführen. Dieser führt zur Koagulation der Eiweißmoleküle wodurch die Konsistenz des Tumorinterstitiums erhöht wird. Diese pH Wert Senkung (bis pH 4) wird u.a. auf die hohe Konzentration an Laktat zurückgeführt, das durch den anaeroben Energiestoffwechsel in den Tumorzellen entsteht. Die Beweglichkeit von Molekülen innerhalb des Interstitiums hängt wesentlich von deren Größe, der Ladung und der chemischen Konfiguration ab. Diese stehen in Wechselbeziehung zu den physiko-chemischen/biochemischen Eigenschaften des Interstitiums.
5. Das Fehlen von funktionellen Lymphgefäßen führt dazu, dass der hydrostatische Druck im Tumor gegenüber dem Normalgewebe stark erhöht ist. Dadurch ist die Konvektion von therapeutisch wirksamen Substanzen entlang der Gefäßwand ins Interstitium sowie innerhalb des Interstitiums stark behindert.
6. Die Effizienz der zellulären Immuntherapie ist durch das Fehlen spezifischer Homing-Rezeptoren durch die unterschiedliche Expression von Adhäsionsmolekülen auf Tumorzellen eingeschränkt und zwar bei verschiedenen Tumorarten und je nach deren Malignitätsgrad in unterschiedlichem Ausmaß.
7. Ein weiterer Aspekt, der es dem Immunsystem erschwert, metastasierende Tumorzellen zu erkennen, wird durch einen spezifischen Mechanismus von metastasierenden Tumorzellen hervorgerufen. Diese maskieren sich, indem sie Marker auf der Zellmembran bzw. Adhäsionsmoleküle reduzieren und so die Erkennung als Fremdantigen erschweren.

Die antitumorale Immunantwort ist prinzipiell in der Lage, Tumorzellen von "normalen" Gewebszellen zu unterscheiden. Allerdings kann das Immunsystem mittels Mechanismen der Toleranzinduktion (wie sie auch für "normale" Zellen bekannt ist) gegenüber Tumorzellen ausbilden, die eine entscheidende Rolle bei der Entstehung der "Resistenz" gegenüber Tumoren spielen können.

Ein wichtiges Indiz für die Effizienz der antitumoralen Immunantwort stellt zum Beispiel die klinische Beobachtung von spontanen (d.h. nicht-Therapie-induzierten) partiellen bis zu kompletten Rückbildungen von Tumoren dar. Im Bereich dieser regressiven Areale finden sich meist leukozytäre Infiltrate, mit einem hohen Anteil an T-Lymphozyten.

Es handelt sich hierbei um spezifisch gegen diesen Tumor gerichtete T-Zellen, die durch eine spezifische Oligoklonalität der tumorinfiltrierenden Lymphozyten (TIL) und der daraus resultierenden spezifischen Fähigkeit, autologe Tumorzellen Antigen-abhängig zu lysieren, charakterisiert sind.

### Beispiele bisher bekannter tumorspezifischer Antigene

Bei den in der Vergangenheit bereits isolierten und charakterisierten Tumorantigenen handelt es sich um:
- Tumorspezifische Antigene (*unique antigens*), die nur in dem entsprechenden Tumor vorkommen. Sie stellen den größten Anteil dar. Sie entstehen durch Punktmutationen oder Rekombinationen von Genen. Zur Tumorvakzinierung werden solche Antigene aus Patientenmaterial, z.B. Biopsiematerial isoliert.
- Antigene, die verschiedenen Tumoren gemeinsam sind (*shared antigens*), aber nicht in normalem Gewebe von Patienten vorkommen. Z.B. sind MAGE-Gene bisher am besten charakterisiert worden. Sie sind in ca. 40% der malignen Melanome nachgewiesen, kommen aber auch im Brust- und Lungentumoren vor. Von ihnen kodieren MAGE-1 und MAGE-3 Gene für Proteine, die von zytotoxischen Lymphozyten erkannt werden.

- Gewebespezifische Antigene, die von entsprechenden Tumoren in wesentlich höherer Anzahl exprimiert werden als im Ursprungsgewebe. Diese können für eine spezifische antitumorale Therapie verwendet werden, ohne eine wesentliche Autoimmunreaktion auszulösen. Diese wurden beispielsweise bei Melanosomen spezifische Proteine (zum Beispiel Mart-1/melan-A oder gp 100) nachgewiesen.
- Weitere Tumorantigene sind z.b. Onkogen- und Tumor-Suppressor-Gen Produkte. Es wurde gezeigt, daß zytotoxische Lymphozyten, die gegen p53 (ein Tumor-Suppressor Protein) gerichtet sind, eine Tumorremission in p53 überexprimierenden Tumoren bewirken können.
- Weiterhin gibt es virusassoziierte Antigene, wie das mit dem humanen Papillomavirus (HPV) assoziierten Zervix-Karzinom oder die Antigene HPV.E6 und E7. Im Kaposi-Sarkomen wurde DNA von HHV-8 (humanes Herpes Virus 8) festgestellt.

Trotz aller Fortschritte in der Aufklärung der Pathogenese humaner Tumoren, sind für die meisten Tumore relevante Tumorantigene oder sog. "Rejectons"-Antigene nicht definiert, zumal die meisten Tumoren keine sog. "shared antigens" sondern individuelle, d.h. tumorspezifische Antigene exprimieren. Erschwerend kommt dazu, dass die biochemische Analyse von tumorspezifischen Antigenen sehr zeitaufwendig, kostenintensiv und z.T. nicht praktikabel ist. Daher werden in den meisten, heute durchgeführten therapeutischen Vakzin-Strategien die Tumorzellen des Patienten nach Isolierung und Manipulation verwendet. Für diese "whole cell"-Ansätze wird in der Regel Biopsiematerial verwendet. Dabei werden die Tumorzellen als Quelle von möglichen (wenn auch nicht näher definierten) "Rejections"-Antigenen verwendet, wobei man davon ausgeht, dass der "pool" von möglichen Antigenen auch relevante Tumor-/Rejections-Antigene enthält.

Ein nicht einfach zu lösendes Problem der "whole cell"-Vakzinierung stellt die Induktion einer Immunantwort gegen sog. Nicht-Tumor-Antigene, also regelhafte "Selbst"-Antigene dar. Die gegen "Selbst"-Antigene gerichtete Immunantwort kann als Risiko für die Auslösung von Autoimmunerkrankungen betrachtet werden. Allerdings geht man davon aus, dass die Immunantwort gegen Tumor-Rejections-Antigene stärker verläuft als gegen Autoantigene.

Die Fähigkeit des Immunsystems, Tumorzellen zu erkennen und diese zu zerstören, wird als "Immunüberwachung" bezeichnet. Es besteht häufig eine Latenzphase bis zur Entwicklung eines "voll"-transformierten Klones (Zwei- oder Mehrschritt-Hypothese der Tumorentstehung). In dieser Latenzzeit der Tumorentstehung kann das Immunsystem Kontakt zu Tumorzellen aufnehmen. Das führt aber trotzdem häufig zu keiner ausreichenden Immunüberwachung. Dieser Zustand wird als friedliche Koexistenz zwischen Tumor und Immunsystem (Toleranz) bezeichnet.

Obwohl offensichtlich eine limitierte Immunantwort vorhanden ist, zeigt das progressive Tumorwachstum, dass das Immunsystem nicht ausreichend kompetent ist.

Dafür gibt es mehrere Ursachen, die für die unzureichende Immunreaktion gegen Tumore verantwortlich sind:
- Einige Tumore exprimieren bestimmte Antigene nicht mehr, regulieren diese herunter oder verlieren sie gänzlich. Das ist die sogenannte "genetische Instabilität" der Tumore.
- Der partielle Verlust der MHC Expression bei Tumoren ist eine andere Möglichkeit für eine mangelhafte Immunreaktion. Durch die Herunterregulation der MHC1 Expression kann der Tumor nicht mehr von zytotoxischen T-Zellen angegriffen und gleichzeitig nicht mehr von NK-Zellen attackiert werden.
- Einige Tumoren sind in der Lage, immunsupprimierende Zytokine zu sezernieren. So kann sich der Tumor vor dem Angriff des Immunsystems schützen. Beispiele dafür sind der "transforming growth factor", der die entzündliche T-Zell-Antwort unterdrückt. Dasselbe gilt auch für IL-10.
- Für die Aktivierung naive T-Zellen sind zwei Signale notwendig. Das erste Signal ist die Kombination aus dem TCR (T-Zell-Rezeptor) und dem jeweiligen Antigen. Das zweite, sog. kostimulierende Signal wird durch die Interaktion des Oberflächenmoleküls B7.1 der APC (Antigen-präsentierenden Zellen) mit dem Oberflächenmolekül CD-28 der T-Zelle vermittelt. Ein alternativer Weg wird durch kostimulierende Signale über Zytokine oder Adhäsionsmoleküle beschritten. Kostimulierende Signale werden beispielsweise im Verlauf einer Entzündung, z. B. auch nach Freisetzung von Zytokinen (Entzündungsmediatoren) von APC exprimiert. Besteht ein Mangel an kostimulierenden Signalen, wenn beispielsweise die Tumorzellen keine Entzündungsreaktion provozieren oder diese mit oben genannten Mechanismen supprimieren, so besteht die Gefahr, dass es statt zur Aktivierung der T-Zellen zu deren lang anhaltender Anergie beziehungsweise zur Apoptose (dem programmierten Zelltod) dieser T-Zellen führen. Somit kann eine periphere Toleranz der T-Zellen gegen den Tumor entstehen, vergleichsweise wie bei der Toleranzentwicklung der T-Zellen gegenüber "normalen" Gewebeantigenen.

Im hier vorgestellten Verfahren erfolgt nach der Isolierung von Tumorzellen aus dem Tumorgewebe, aus dem Blut oder den Lymphknoten des Patienten die Eröffnung der Zellmembran der Tumorzellen, die zur einfacheren Identifizierung zuvor durch einen geeigneten Fluoreszenzfarbstoff markiert werden können. Die Eröffnung erfolgt mittels eines Ultrakurz-Laserimpulses, so dass natives Zellmaterial freigesetzt wird und die native Zellmembran komplett freiliegt. Das native Zellmaterial stellt den Antigenpool dar, indem sich ein komplettes Arsenal tumorspezifischer Antigene befindet. Die native aufgeschnittene Zellmembran dient den APC als kostimulierender Faktor. Sie enthält sowohl Tumorantigene, wie beispielsweise Adhäsionsmoleküle und transmembranale Rezeptoren, die bei den Tumorzelle in unterschiedlicher Weise exprimiert werden können, als auch Phospholipidanteile bzw. Lipoproteine und Proteoglykane, die als Entzündungsmediatoren wirken. Da die gewonnenen Antigene als auch die kostimulierenden Faktoren nativ vorliegen, also nicht chemisch behandelt oder durch physikalisch bedingten Energieeintrag in ihrer Struktur modifiziert wurden bzw. ihre chemische Konformität beeinflusst wurde, können sie die antitumorale Immustimulanz am effektivsten induzieren. Da der komplette spezifische tumorale Antigencocktail in unveränderter Form vorliegt, ist zu erwarten, dass auch das Risiko einer autoimmunen Reaktivität minimiert wird.

Nach der Gewinnung der Tumorantigene und der kostimulierenden Faktoren werden diese in einer Reaktionskammer mit den aus dem Patientenblut gewonnenen Immunzellen kokultiviert und anschließend wieder dem Patienten infundiert. Zuvor wird anhand eines Tests über ein Zytokinpanel das Risiko einer autoimmunologischen Reaktion ermittelt.

Der Überstand des aus dem Biopsiematerial, dem Blut oder den Lymphknoten des Patienten gewonnenen tumoralen Antigenmaterials und der kostimulierenden Faktoren, kann ggf. später für eine Boosterung verwendet werden.

### Immuntoleranz

Die potentesten Antigen-präsentierenden Zellen, sind dendritische Zellen, die durch erkannte Fremdantigene aktiviert werden, um eine Immunantwort zu erzeugen. Dendritische Zellen werden durch Fremdantigene häufig nicht ausreichend aktiviert, so dass konservierte Moleküle von Bakterien oder auch Viren als "Adjuvanzien", benötigt werden. Es gibt auch die Möglichkeit durch endogene Aktivatoren dendritische Zellen zu aktivieren, wie sie im Rahmen von Entzündungen als Entzündungsmediatoren (z.B. Tumornekrosefaktor alpha und aktivierte T-Zellen) vorkommen. Diese werden im Rahmen einer Entzündungsreaktion generiert. Für deren Initiierung benötigt man aber zunächst die aktivierte Form Antigenpräsentierender Zellen, d.h. diese Faktoren wirken lediglich im Sinne eines positiven Feedback-Mechanismus und weniger als Initiation der Immunantwort. Direkt wirkende endogene Signale für die Aktivierung von dendritischen Zellen sind u.a. zellulärer Stress oder bestimmte Signale, die von nekrotischen, nicht apoptotischen, also nicht dem programmierten Zelltod unterliegende, absterbenden oder toten Zellen ausgehen, sowie Zytokine, die z.B. nach Virusinfektion von Zellen produziert werden. Vitale Zellen oder Zellen, die im programmierten Zelltod (Apotose) sterben, produzieren hingegen solche endogene Aktivatoren dendritischer Zellen nicht. Diese endogenen aktivierenden Substanzen werden als natürliche Adjuvanzien bezeichnet, die eine primäre Immunantwort stimulieren. Sie sind natürliche Initiatoren beispielsweise der Transplantatabstoßung, aber auch der spontanen Tumorabstoßung und auch für verschiedene Formen von Autoimmunerkrankungen.

Ruhende dendritische Zellen sind also prinzipiell in der Lage, apoptotische und nekrotische Zellen gleichermaßen zu phagozytieren. Eine wichtige Einschränkung dabei ist, dass diese dendritischen Zellen von MHC und kostimulierenden Molekülen von nekrotischen Zellen, nicht jedoch von apoptotischen Zellen induziert werden. Dieses Phänomen stellt ein wichtiges Hemmnis für die Stimulierung dendritischer Zellen dar, da die meisten Tumorzellen unter ungünstigen Überlebensbedingungen, beispielsweise bei einer Chemotherapie oder Strahlentherapie, den Vorgang der Apoptose, also des programmierten Zelltodes, einleiten.

Zur Erklärung der Phänomene der antitumoralen Immunantwort kann man die Immuntoleranz gegenüber einem Fetus heranziehen.

Das Fehlen einer starken Immunantwort gegenüber Tumoren (vergleichbar der Reaktion gegenüber dem Fetus) wird durch die Tatsache erklärt, dass es sich sowohl bei Feten als auch bei Tumoren um vitale Gewebe handelt, die damit keine ausreichenden Stress-Signale bzw. kostimulierende Signale im Zusammenhang mit der Antigen-Expression aufweisen. Zusätzlich sind noch supprimierende Faktoren wie Zytokine oder blockierende Liganden relevant. So kann beispielsweise die Aktivierung von CD40 über löslichen CD40- Liganden oder über aktivierende CD40-Antikörper zur Überwindung einer möglichen peripheren T-Zell-Toleranz führen.

Dendritische Zellen werden in drei distinkte Subpopulationen eingeteilt. Zwei lassen sich aus der myeloischen Reihe ableiten, nämlich die Langerhans-Zellen und die interdigitierenden Retikulumzellen oder interstitiellen dendritischen Zellen. Die dritte Subpopulation leitet sich aus dem lymphatischen Zellsystem ab. Es handelt sich dabei um CD11c negative, CD4 und CD68 positive, plasmazytoide T-Zellen bzw. plasmazytoide Monozyten, die im Gegensatz zu den myeloischen dendritischen Zellen nicht GMCSF, sondern auf IL-3 oder Monozytenkonditioniertem Medium überleben und differenzieren.

Dies sind die sogenannten PDC2/IPC-Zellen, die stärkste bekannteste Population natürlicher Interferon-produzierender Zellen (IP10). Diese Zellen exprimieren darüber hinaus ILT3 (Immunglobulin-ähnlicher Rezeptor), allerdings sind sie SILT1 negativ. Die induzierte Interferonproduktion erfolgt entweder durch Kontakt mit Viren oder virusinfizierten Zellen oder durch CD40-Aktivierung auf diesen Zellen. PDC2 Zellen können T-Zellen dazu induzieren, IL-4 und eine T-Helfer-2-Antwort zu initiieren.

Am Anfang der Aktivierung der PDC2-Zellen steht die hohe Interferon-Produktion Damit wird das unspezifische angeborene Immunsystem aktiviert. Durch die nachfolgende Induktion von T-Zellen und dem T-Helfer-2-Zytokin wird die Antigen-spezifische adaptive Immunantwort getriggert. Auf diese Weise werden nicht nur die Pathogene eliminiert, wie z.B. die Elimination des Virus durch Hemmung der Virusreplikation durch Aktivierung von NK-Zellen und Makrophagen, sondern auch durch die Hochregulation von MHC1-Molekülen und die erhöhte Suszeptibilität gegenüber zytotoxischen T-Lymphozyten. Es wird auch das Überleben von B- und T-Zellen gesteigert, wodurch über die Ausreifung von CD4-positiven T-Helfer-Zellen eine Induktion von Antikörper-sezernierenden B-Zellen erfolgt. Darüber hinaus erfolgt durch die T-Helfer-2-Aktivierung ein negatives Feedback, das wiederum zur Herunterregulation der TH1-Immunantwort, zum Beispiel durch die supprimierenden Mediatoren IL-4 und IL-10 führt.

Die Toleranzentstehung des Immunsystems gegenüber Tumoren ist dem Prinzip der Toleranzentwicklung bei Transplantaten ähnlich. Die Interaktion von T-Zellen und Antigen-präsentierenden Zellen wird über eine Reihe von Oberflächenmolekülen gesteuert. Als erstes ist der Kontakt des T-Zell-Rezeptors mit dem Antigen-MHC-Komplex im Sinne des Primärsignals, als auch die Ko-Stimulation der T-Zellen für eine T-Zellaktivierung notwendig. Das zweite Signal, das sogenannte kostimulierende Signal wird in der Regel durch die Interaktion von CD40-Ligand und CD28 auf den T-Zellen, sowie CD40 und CD80 (B7.1) auf der Oberfläche der Antigen-präsentierenden Zellen generiert. Beim Fehlen dieses zweiten, kostimulierenden Signals kommt es zur Anergie der T-Zellen gegenüber dem präsentierenden Antigen, also zur Toleranz. Es existiert die Hypothese, dass nur dendritische Zellen in der Lage sind, naive T-Zellen zu stimulieren, während Memory-T-Zellen auch durch andere Antigen-präsentierende Zellen aktiviert werden können, wie beispielsweise B-Zellen, Makrophagen oder Endothelzellen. Man weiß allerdings mittlerweile, dass dendritische Zellen zu aktivierten Antigen-präsentierenden Zellen differenzieren, wenn sie ihrerseits entsprechende stimulierende Signale, u.a. über eine Aktivierung ihres Oberflächenmoleküls durch den CD40-Liganden empfangen.

Für die effiziente Immuntherapie von Tumoren sind deshalb folgende Schritte notwendig:
1. Induktion des Erkennungsprozesses der Tumore durch das Immunsystem.
2. Ausschaltung der Toleranz des Immunsystems.
3. Gezielte Stimulation und gerichtete Manipulation des Immunsystems, also der T-Zellen und der Antigen-präsentierenden Zellen gegen Tumorzellen.
4. Vermeidung der Aktivierung des Immunsystems gegen körpereigene gesunde Zellen.

Tumore können also auf verschiedene Weise der Immunüberwachung entgehen. Deshalb sind die Verstärkung der Immunogenität eines Tumors und/oder die Verstärkung der Immunantwort durch gezielte Manipulation des Immunsystems, Schlüsselmechanismen und stellen eine der wichtigsten Voraussetzungen für eine erfolgreiche Anti-Tumor-Therapie dar.

Die Beeinflussung des Immunsystems und Erzeugung einer gegen den Tumor gerichteten effektiven Immunantwort im Zusammenhang mit dem Unterdrücken von Mechanismen wie Toleranz, Ignoranz, Anergie oder negative Selektion gegenüber von Tumorzellen bei gleichzeitiger Vermeidung von Risiken von autoimmunologischer Fehlreaktion ist das Ziel dieser Erfindung.

Bei viralen oder bakterielle Infektionen kommt es typischerweise zu einer Aktivierung des Immunsystems, während eine "endogene" Exposition von Antigenen ohne entsprechende Entzündungsmediatoren üblicherweise eine immunologische Toleranz bewirkt.

Bei der aktiven Immunisierung gegen virale oder bakterielle Erreger mittels Vakzinierung werden Adjuvanzien, also "Hilfsmittel" beigegeben, um die Effektivität der Vakzinierung zu verbessern. Ihre Wirkungsweise bietet aber leider auch Risiken, deren Ursachen bisher häufig ungeklärt sind, da sie oft auf empirischen Untersuchungen basieren.

### Konventionelle Ansätze der Tumorvakzinierung

Die Tumorvakzine sollten so modifiziert sein, dass sie selbst keinen Tumor erzeugen, jedoch eine ausreichende Immunogenität auslösen. Die Vakzine selbst sollte im Idealfall lediglich subklinische Krankheitsbeschwerden ohne signifikante Erkrankungen auslösen. Bestrahlte Tumorzellen können sich im Körper idealerweise nicht mehr vermehren, sind jedoch nur noch ca. 2 Wochen lebensfähig, so dass eine ausreichende Antigenpräsentation des Immunsystems erreicht wird, mit dem Ziel, eine protektive Immunität gegen spezifische "Tumorantigene" zu erreichen. Das kann man prinzipiell auch durch Verwendung abgetöteter Zellen (eingefroren, lysiert, erhitzt) erreichen. Allerdings sind diese Antigene durch den Vorgang der Lyse, in der Regel entweder denaturiert oder zu mindestens in ihrer chemischen Struktur und damit ihrer Konformität so verändert, dass nur eine ineffiziente, größtenteils zufällige Antigenerkennung möglich ist.

Diese sogenannten "Rejections-Antigene" sollten prinzipiell diejenigen Antigene sein, mit denen der Wirt bereits während der Tumorentstehung Kontakt hatte und die zu einer Anergie oder Toleranz des Immunsystems führen. Allerdings sind Modifikationen durch Mutationen oder der Verlust im Verlauf der Tumorentwicklung möglich. Ziel von immuntherapeutischen Strategien muss es sein, diese Toleranz, die von unterschiedlicher Qualität und Quantität sein kann, in jedem Fall zu durchbrechen. Eine Möglichkeit dies zu erreichen ist die Erzeugung einer Immunantwort auf Basis einer "Wieder"- Auseinandersetzung mit dem Tumor bzw. den Tumorantigenen durch "gerichtete" Induktion einer Entzündung.

Die Erfindung verfolgt die Möglichkeit, eine Entzündung durch die Kostimulation mit den nativen Teilen der eröffneten Zellmembran und den freigelegten Strukturproteinen zu induzieren. Der darin u.a. enthaltene Cocktail verschiedener Glykosphingolipide und verschiedener Glycopolysachharide, Proteoglycane etc. triggert die Immunantwort als kostimulierender Faktor über eine induzierte Entzündung, die eine Toleranzreaktion unmöglich macht. Damit führt die Immunantwort zur Bildung von Effektor- und Memoryzellen. Die wiederholte Kokultivierung neuer Immunzellen mit dem Lysat erzeugt eine gesteigerte und schnellere Immunantwort (die sog. Sekundär-Antwort).

Die oben beschriebenen Tumorvakzinierungsstrategien zielen darauf ab, ähnlich einer Vakzinierung gegen fremde Krankheitserreger, eine generelle (für verschieden Patienten gleich gestaltete) Immunantwort gegen eine Tumorentität zu generieren. Im Vergleich dazu stellt das von uns erfundene Verfahren eine stimulierte, individuelle Immunantwort für den speziellen Tumor des Patienten dar.

### Wirkung von Ultrakurz-Laserimpulsen

Unter optischen Ultrakurzpulsen versteht man elektro-magnetische Wellenzüge im optischen Wellenlängenbereich mit einer zeitlichen Breite im Pikosekunden- (ps) oder Femtosekundenbereich (fs). Erzeugt werden können solche Pulse in speziellen Lasern (Ultrakurzpuls-Laser) durch verschiedene Impulsverkürzungstechniken, die eine Gleichphasigkeit aller axialer Moden im Resonator erzeugen (Modenkopplung).

Im Unterschied zu längeren Pulsen wird die Energie solcher Ultrakurzpulse, wenn die Wechselwirkung mit Molekülen zu einer Absorption führt, nur auf einen sehr begrenzten Radius verteilt (Energiedissipation). Ursache dafür ist die extrem kurze Einwirkzeit. Das wiederum führt zu einer sehr hohen Energiedichte im Bereich des Fokus, wodurch selbst mit einer sehr geringen mittleren Energie Molekülbindungen aufgetrennt werden können.

Im Fall der Fokussierung auf eine Zellmembran trennt der Impuls Verbindungen innerhalb der Membran ohne die Moleküle der Membran wesentlich zu verändern oder eine ausgedehnte thermische Energiedissipation innerhalb der Membran zu verursachen.

Über eine genau definiert Anzahl dieser Impulse, die örtlich über die Membran verteilt werden, kann die Zellmembran über eine bestimmte Strecke schonend aufgetrennt werden. Aufgrund der äußerst geringen thermischen Energiedissipation ist die molekulare Zerstörung der Membran so gering, dass sie vernachlässigt werden kann.

Werden Ultrakurzpulse zur Eröffnung bzw. Lyse von Zellen verwendet ergibt sich der wesentliche Vorteil gegenüber den konventionellen Methoden (vgl. 3.1) aus den o.g. Gründen. Im Gegensatz zu den genannten Methoden erfolgt der Energieeintrag lediglich auf einem stark begrenzten, vernachlässigbar kleinen Volumen auf der Zellmembran/-wand.

Daraus folgt, dass lediglich die strukturelle Integrität der Zellmembran aufgelöst wird, die Bestandteile der Membran selbst aber unbeeinflusst bleiben. Darüber hinaus gibt es keine thermische oder mechanische Beeinflussung und keine Zerstörung der Zellbestandteile (Organellen, Struktur- und Funktionsproteine etc.).

### Blockschema des neuen Verfahrensansatzes

| **Schritt** | **Methode** | **Vorteile** | **Effekt** |
|---|---|---|---|
| 1 | Gewinnung von Tumorzellen des Patienten aus Biopsiematerial oder von in der Blut- oder Lymphbahn zirkulierenden Tumorzellen | • **Gewinnung von originären Tumorzellen des Patienenten** | Gewinnung von Tumorzellen im nativen Gewebe im unveränderten Zustand |
| | | **→ ermöglicht eine individuelle antitumorale Immunantwort** | |
| 2 | Eröffnung der Tumorzelle mittels Ultrakurzzeitlaser Impulse | • **Keine Modifikation der Moleküle** | Freisetzung von antitumoralen immunstimulierenden Faktoren |
| | | • **Ein vollständiges Panel an individuellen, tumorspezifischen Tumorantigenen** | **a. ein Panel von individuellen, tumorspezifischen Antigenen** |
| | | **→ ermöglicht eine hocheffiziente individuelle antitumorale Immunantwort** | verschiedene, chemisch und physikalisch unveränderte Moleküle |
| | | • **Bestandteile der Zellmembran als immunogenen costimulierende Faktoren** | • des Zytoplasmas |
| | | | • der Zellorganelle |
| | | | • der Zellmembran (z.B. Rezeptormoleküle) |
| | | **→ ermöglicht eine hocheffiziente Verstärkung der antitumoralen Immunantwort** | **b. immunogenen costimulierende Faktoren** |
| | | | verschiedene, chemisch und physikalisch |
| | | • **die morphologische Zellstruktur bleibt intakt** | unveränderte Moleküle der Zellmembran |
| | | | **c. Minimierung der Apotosen der Tumorzellen** |
| | | **→ Reduktion der Gefahr einer Apoptose der Tumorzelle** | die Unterdrückung der antitumoralen Immun-durch |
| | | | Apoptose der Tumorzelle wird vermieden |
| 3 | Gewinnung von Immunzellen aus dem Blut des Patienten | | |
| 4 | Kokultivierung des im Schritt 2 gewonnenen antigenen Materials und den immumnogen, costimulierenden Faktoren mit den im Schritt 3 erhaltenen Immunzellen des Pateinten | **• Bei der Kokultivierung der Immunzellen mit dem nicht veränderten tumoralen Antigenpanel wird die Spezifität und Effektivität der Immunantwort immens gesteigert.** | Durch die Kokultivierung der gewonnenen Immunzellen mit dem unveränderten Antigenpanel des spezifischen, individuellen Tumors des Patienten und den gewonnenen immunogen costimuliernden Faktoren wird über die verschiedenen Immunzellen eine Tumor-spezifische und hocheffektive Immunantwort induziert. |
| | | **• Eine Unterdrückung der Immunantwort durch immunsuppressive Mechanismen, die durch den Tumor normalerweise induziert werden, wird so außer Kraft gesetzt.** | |
| 5 | Testung der Effizienz antitumoralen Immunantwort | **• Die Behandlung kann wiederholt werden.** | **a. Durch die Gewinnung von antituroralen Immunmarkern aus dem Überstand der Kokultur, kann der Erfolg der Immunantwort gemessen und verifiziert werden.** |
| | | **• Beim Auftreten von immunbedingten Komplikationen kann die Behandlung frühzeitig abgebrochen werden.** | **b. Durch Ermittlung von immunologischen Markern aus dem Überstand der Kokultur, kann eine Fehlreaktion des Immunsystems erkannt werden (z.B. Autoimmunität).** |
| 6 | Reinfusion der Kokultivierten Immunzellen des Patienten | | |
| 7 | Nachkontrolle des Behandlungserfolges und ggf. Wiederholung der Behandlung | | |

## Patentansprüche

1. Verfahren zur Aktivierung von aus Patientenblut gewonnenen Immunzellen zur Stimulation einer antitumoralen körpereigenen Immunantwort,
**gekennzeichnet durch** die Schritte:
- Perforieren wenigstens einer Zellmembran wenigstens einer Tumorzelle mittels eines Ultrakurzpuls-Lasers zur Gewinnung von nativen Tumorantigenen und Teilen der perforierten Zellmembran;
- Kokultivieren wenigstens einer von aus Patientenblut gewonnen Immunzellen mit den nativen Tumorantigenen und den Teilen der wenigstens einen perforierten Zellmembran.

2. Verfahren nach Anspruch 1, **dadurch gekennzeichnet, dass** der Schritt des Perforierens eine optische Perforation der Zellmembran umfasst.

3. Verfahren nach Anspruch 1 oder 2, **dadurch gekennzeichnet, dass** bei dem Schritt des Perforierens die Parameter der Laserimpulse des Ultrakurzpuls-Lasers derart eingestellt werden, dass eine Veränderung der chemischen Molekülstruktur der Tumorantigene vermieden wird.

4. Verfahren nach einem der Ansprüche 1 bis 3, **dadurch gekennzeichnet, dass** vor dem Schritt des Perforierens die Tumorzellen zur Identifizierung mittels eines Fluoreszenzfarbstoffs angefärbt werden.

5. Verfahren nach einem der Ansprüche 1 bis 4, **dadurch gekennzeichnet, dass** der Schritt des Kokultivierens ein Verwenden der der Tumorantigene und der Teile der perforierten Zellmembran als kostimulierende Antigene umfasst.

6. Verfahren nach einem der Ansprüche 1 bis 5, **dadurch gekennzeichnet, dass** nach dem Schritt des Kokultivierens die derart aktivierten Immunzellen für eine effiziente Stimulierung der antitumoralen körpereigenen Immunantwort genutzt werden.

7. Verfahren nach einem der Ansprüche 1 bis 6, **dadurch gekennzeichnet, dass** während und nach dem Schritt des Kokultivierens eine begleitende Effizienzkontrolle der Qualität und der Quantität der Immunstimulation durchgeführt wird.

8. Verfahren nach Anspruch 7, **dadurch gekennzeichnet, dass** die Effizienzkontrolle der Immunantwort Aufschluss über eine mögliche fehlerhafte Immunantwort (Autoimmunität) gibt.

9. Verfahren nach einem der Anspruch 1 bis 8, **dadurch gekennzeichnet, dass** das mittels des Ultrakurzpuls-Laser-Verfahrens nativ gewonnene antigene Material für spätere Wiederholungsanwendungen zur Erzielung eines Booster-Effekts verwendet wird.
